# EUROPÄISCHE PATENTSCHRIFT

(11) **EP 2 358 758 B1**
(45) Veröffentlichungstag und Bekanntmachung des Hinweises auf die Patenterteilung: **05.09.2012**
(21) Anmeldenummer: 08875391.8
(22) Anmeldetag: 28.11.2008
(51) Int. Cl.: C08B 37/00, A61L 27/52

(54) **BIOAKTIVES HYDROGEL**
BIOACTIVE HYDROGEL
HYDROGEL BIOACTIF

(43) Veröffentlichungstag der Anmeldung: 24.08.2011
(73) Patentinhaber: Zetascience GmbH, 01307 Dresden (DE)
(72) Erfinder: WERNER, Carsten, 01069 Dresden (DE); FREUDENBERG, Uwe, Dr., 01328 Dresden (DE); MEINHOLD, Dorit, 01109 Dresden (DE); GOUZY, Marle-Francoise, 4123 Allschwill (CH); WELZEL, Petra, 01109 Dresden (DE)
(74) Vertreter: Sperling, Thomas
(86) Internationale Anmeldenummer: PCT/EP2008/066484
(87) Internationale Veröffentlichungsnummer: WO 2010/060485

(56) Entgegenhaltungen:
- WO-A-2006/034467
- WO-A-2007/127198
- WO-A-2008/108736
- YAMAGUCHI N ET AL: "Polysaccharide-poly(ethylene glycol) star copolymer as a scaffold for the production of bioactive hydrogels" BIOMACROMOLECULES JULY/AUGUST 2005 AMERICAN CHEMICAL SOCIETY US, Bd. 6, Nr. 4, Juli 2005 (2005-07), Seiten 1921-1930, XP002539059
- ZHANG L ET AL: "Manipulation of hydrogel assembly and growth factor delivery via the use of peptide-polysaccharide interactions" JOURNAL OF CONTROLLED RELEASE, ELSEVIER, AMSTERDAM, NL, Bd. 114, Nr. 2, 28. August 2006 (2006-08-28), Seiten 130-142, XP024957584 ISSN: 0168-3659 [gefunden am 2006-08-28]
- NIE ET AL: "Production of heparin-functionalized hydrogels for the development of responsive and controlled growth factor delivery systems" JOURNAL OF CONTROLLED RELEASE, ELSEVIER, AMSTERDAM, NL, Bd. 122, Nr. 3, 18. September 2007 (2007-09-18), Seiten 287-296, XP022336560 ISSN: 0168-3659
- SEAL ET AL: "Physical matrices stabilized by enzymatically sensitive covalent crosslinks" ACTA BIOMATERIALIA, ELSEVIER, AMSTERDAM, NL, Bd. 2, Nr. 3, 1. Mai 2006 (2006-05-01), Seiten 241-251, XP005394598 ISSN: 1742-7061
- ZHANG ET AL: "Preparation and characterization of <99m>Tc(CO)3-BPy-RGD complex as alphavbeta3 integrin receptor-targeted imaging agent" APPLIED RADIATION AND ISOTOPES, ELSEVIER, OXFORD, GB, Bd. 65, Nr. 1, 9. November 2006 (2006-11-09), Seiten 70-78, XP005755102 ISSN: 0969-8043

## Beschreibung

Die Erfindung betrifft ein bioaktives Hydrogel, welches als Biomaterial für den Ersatz von biologischem Gewebe, als Implantatmaterial oder im weitesten Sinne in Medizinprodukten einsetzbar ist.

Unter einem Biomaterial im Sinne der Erfindung sollen Materialien verstanden werden, die mit einem biologischen Organismus bei diagnostischen oder therapeutischen Anwendungen in Kontakt gebracht werden. Diese Materialien müssen spezielle Anforderungen im Hinblick auf die Biokompatibilität erfüllen. Unter Biokompatibilität ist das Ausbleiben klinisch signifikanter Reaktionen des Organismus auf den Einsatz von Materialien, Medizinprodukten oder medizinischen Systemen zu verstehen.

Gattungsgemäße bioaktive Hydrogele werden mit besonderem Vorteil als Implantat- oder Gewebsersatzmaterialien eingesetzt.

Im Stand der Technik sind diverse Ansätze zur Erzeugung von bioaktiven Hydrogelen bekannt. Es wurden verschiedene Implantat- oder Gewebsersatzmaterialien zum Einsatz in regenerativen Therapien erforscht, beispielsweise zur Unterstützung der Regeneration von Blutgefäßen und Nervenbahnen oder als Hautersatzmaterialien. Hierfür wurden Gerüst- oder Trägermaterialien, so genannte Scaffolds, auf Basis biologischer oder synthetischer Hauptkomponenten entwickelt, welche nach der Transplantation temporär wichtige Funktionen der natürlichen Extrazellulären Matrix EZM erfüllen sollen. Zellen in natürlichen Geweben existieren innerhalb dieser EZM. Die EZM ist ein komplexes, supramolekulares Netzwerk aus verschiedenen Strukturproteinen, hauptsächlich Kollagen, Proteoglykanen, Glykoproteinen und Elastin, dessen struktureller Aufbau und funktionelle Zusammensetzung für die Aufrechterhaltung der normalen Gewebearchitektur sowie für gewebsspezifische Funktionen essentiell ist.

Nach dem derzeitigen Stand der Wissenschaft werden für die oben angesprochenen regenerativen Prozesse so genannte Scaffolds angewendet, die für die für Regenerationsprozesse relevanten Zellen primär eine Träger-und Stützfunktion wahrnehmen und den Schutz vor mechanischer Belastung gewährleisten.

Aus der US 6,306,922 A und der US 6,602,975 ist beispielsweise bekannt, stark hydratisierte Materialen, so genannte Hydrogele aus synthetischen oder biologischen Hauptkomponenten, einzusetzen, welche über längere Zeiträume ohne zellschädigende Wirkung im Körper abbaubar sind.

Um auch komplexe multizelluläre Prozesse unterstützen zu können, wurden hierfür auch Mischungen aus Komponenten der natürlichen EZM oder auch Materialien zur reversiblen Bindung und Freisetzung therapeutisch relevanter Signalmoleküle entwickelt. Für die letztgenannten Funktionen wurden auch Kombinationen aus synthetischen und polysacharidbasierenden Komponenten der natürlichen EZM entwickelt, welche die besondere Affinität dieser Moleküle zu wichtigen Signalmolekülen, beispielsweise Wachstumsfaktoren, ausnutzen.

Aus Yamaguchi N et al: "Polysaccharide-poly(ethylene glycol) star copolymer as a scaffold for the production of bioactive hydrogels" BIOMACROMOLECULES JULY/AUGUST 2005 AMERICAN CHEMICAL SOCIETY US, Bd. 6, Nr. 4, Juli 2005 (2005-07), Seiten 1921-1930, XP002539059' und aus ZHANG L ET AL: "Manipulation of hydrogel assembly and growth factor delivery via the use of peptide-polysaccharide interactions" JOURNAL OF CONTROLLED RELEASE, ELSEVIER, AMSTERDAM, NL, Bd. 114, Nr. 2, 28. August 2006 (2006-08-28), Seiten 130-142, XP024957584 ISSN: 0168-3659' sowie aus SEAL ET AL: "Physical matrices stabilized by enzymatically sensitive covalent crosslinks" ACTA BIOMATERIALIA, ELSEVIER, AMSTERDAM, NL, Bd. 2, Nr. 3, 1. Mai 2006 (2006-05-01), Seiten 241-251, XP005394598 ISSN: 1742-7061 sind die nicht-kovalente Bindung von Polyethylenglykol und Heparin bekannt.

In NIE ET AL; "Production of heparin-functionalized hydrogels for the development of responsive and controlled growth factor delivery systems" JOURNAL OF CONTROLLED RELEASE, ELSEVIER, AMSTERDAM, NL, Bd. 122, Nr. 3, 18. September 2007 (2007-09-18), Seiten 287-296, XP022336560 ISSN: 0168-3659 sind Heparin und Polyethylenglykol über eine Michael-Reaktion von SH-funktionalisiertem PEG und Heparin gebunden.

Weiterhin geht aus der WO 2006/034467 A (JULIAZ INC [US]; HNOJEWYJ OLEXANDER [US]) 30. März 2006 (2006-03-30) ein aktiviertes PEG und eine Komponente enthaltendes Heparin und ein aminofunktionelles PEG hervor.

Die WO 2007/127198 A (INCEPT LLC [US]; PATHAK CHANDRASHEKHAR P [US]; SAWHNEY AMARPREET S [US]) 8. November 2007 (2007-11-08) betrifft ein Gel, erhalten aus einem PEG, Heparin und Hydroxylaminsuccinat NHS-Ester.

Die WO 2008/108736 A (AGENCY SCIENCE TECH & RES [SG]; YING JACKIE Y [SG]; WAN ANDREW C A [SG]) 12. September 2008 (2008-09-12)' offenbart Heparin und Polyethylenglykol, welche über eine Michael-Reaktion von SH-funktionalisiertem Heparin und PEG-Vinylsulfon gebunden sind.

Nachteilig an den im Stand der Technik bekannten Materialien ist, dass die komplexen therapeutischen Aufgaben von den bekannten Hydrogelen nicht in allen Belangen ausreichend effektiv gelöst werden.

Außerdem sind die meisten bisher beschriebenen Materialien aufgrund limitierter Vemetzungschemie oder der ausschließlichen Verwendung von Naturstoffen in ihren physikalischen, insbesondere in ihren mechanischen Eigenschaften in Bezug auf Steifigkeit und Quellung auf einen engen Bereich eingeschränkt.

Die Aufgabe der Erfindung besteht darin, ein stark hydratisiertes und gelartiges Material mit gezielt abstufbaren physikalischen und biochemischen

Eigenschaften und ein Verfahren zur Herstellung eines solchen Materials zur Verfügung zu stellen.

Das Material soll dabei langfristig im Körper ohne toxische Spaltprodukte abbaubar und biokompatibel sein.

Darüber hinaus sollte das Material eine Möglichkeit bieten, alle wichtigen Funktionen der natürlichen Extrazellulären Matrix modular, dass heißt weitgehend unabhängig voneinander, abzubilden.

Die sich daraus ableitenden Aufgaben sind im Einzelnen:
- die Gerüst-, Stütz- und Schutzfunktion für einwachsende Zellen spezifisch, je nach Anwendungsgebiet, zu verbessern,
- die Kontrolle der Zelladhäsion zu ermöglichen,
- die reversible Bindung und Freisetzung therapeutisch relevanter Signalmoleküle zu ermöglichen und
- die Möglichkeit des bedarfsgerechten Umbaus durch einwachsende Zellen zu schaffen.

Die Aufgabe der Erfindung wird durch ein bioaktives Hydrogel als Hybridmaterial aus Heparin und sternförmig verzweigtem endgruppenfunktionalisierten Polyethylenglykol gelöst, wobei das Heparin direkt durch 1-Ethyl-3-(3-dimethylaminopropyl) carbodiimid/N-Hydroxysulfosuccinimid (EDC/s-NHS) aktivierte Carboxylgruppen des Heparins mit den endständigen Aminogruppen des Polyethylenglykols kovalent durch Amidbindungen verbunden ist.

Alternativ wird die Aufgabe durch ein bioaktives Hydrogel als Hybridmaterial aus Heparin und sternförmig verzweigtem endgruppenfunktionalisierten Polyethylenglykol gelöst, wobei das Heparin über kurze enzymspaltbare Peptidsequenzen als Vernetzungsmolekül kovalent mit dem Polyethylenglykol verbunden ist.

Hierbei sind zwei Wege möglich:

Der erste Weg verläuft über die Funktionalisierung des PEGs, wobei die Funktionalisierung des Polyethylenglykols mit enzymspaltbaren Peptidsequenzen durch Reaktion der mit 1-Ethyl-3-(3-dimethylaminopropyl) carbodiimid/N-Hydroxysulfosuccinimid (EDC/s-NHS) aktivierten Carboxylgruppe am C-Terminus des Peptides mit den Aminogruppen des Polyethylenglykols erfolgt, und im Anschluss erfolgt die eigentliche Gelbildung durch Umsetzung der Aminogruppe am N-Terminus des mit dem PEG verbundenen Peptides mit den mit 1-Ethyl-3-(3-dimethylaminopropyl) carbodiimid/N-Hydroxysulfosuccinimid (EDC/s-NHS) aktivierten Carboxylgruppen des Heparins.

Der zweite Weg verläuft über die Funktionalisierung des Heparins, wobei die Funktionalisierung des Heparins mit enzymspaltbaren Peptidsequenzen durch Reaktion der mit 1-Ethyl-3-(3-dimethylaminopropyl) carbodiimid/N-Hydroxysulfosuccinimid (EDC/s-NHS) aktivierten Carboxylgruppen des Heparins mit der Aminogruppe am N-Terminus des Peptides erfolgt, und im Anschluss die eigentliche Gelbildung durch Umsetzung des mit den Peptiden funktionalisierten Heparins erfolgt, indem die durch 1-Ethyl-3-(3-dimethylaminopropyl) carbodiimid/N-Hydroxysulfosuccinimid (EDC/s-NHS) aktivierte Carboxylgruppe am C-Terminus des Peptides mit den Aminogruppen des PEGs reagiert.

Nebengeordnete Ausgestaltungen der Erfindung bestehen darin, dass eine Folie aus den erfindungsgemäßen Hydrogelen zur Verfügung gestellt wird, welche erhältlich ist durch
- Auftropfen einer definierten Menge flüssiger Gelmaterialien auf hydrophobierte Trägerflächen, insbesondere Deckglas oder Siliciumwafer,
- Gelbildung nach dem Abdecken der Trägerflächen mit hydrophobiertem Glasträger oder Siliciumwafer,
- Überführen der Trägerflächen in eine Waschlösung und
- Entfernung der Folien nach dem Quellen der Hydrogele.

Die erzeugbaren Folien weisen in bevorzugten Ausgestaltungen eine Dicke von 80 bis 2000 µm auf, wobei auch Folien mit Dicken von mehreren Millimetem aus diesem Material und nach dem angegebenen Verfahren erzeugbar sind.

Als Waschlösung wird vorteilhaft PBS eingesetzt.

Eine weitere, sich aus der Erfindung ergebende Anwendung besteht darin, dass hohlzylinderförmige, tubuläre Formkörper aus erfindungsgemäßen Hydrogelen mit einer Länge von bis zu 7 cm und einem Innendurchmesser von 0,1 bis 0,8 mm herstellbar sind.

Die tubulären Strukturen werden beispielsweise dadurch erhalten, wenn in einen kapillarförmigen Schlauch aus rekonstituierter Zellulose noch flüssiges Hydrogel injiziert und anschließend ein Platzhalter in den Schlauch eingeführt wird, wonach das bioaktive Hydrogel gebildet und der Platzhalter im Anschluss entfernt wird.

Eine weitere Anwendung der Erfindung besteht in einem Zellkulturträger mit einem erfindungsgemäßen Hydrogel, welcher dadurch gekennzeichnet ist, dass das Hydrogel kovalent über reaktive Polymerdünnschichten aus alternierenden MSA-Copolymeren angekoppelt wird, wobei die Gelbildung in Gegenwart der Anhydridgruppen enthaltenden polymerbeschichteten anorganischen Träger durchgeführt wird und die Hydrogele über die Aminogruppen des sternförmigen Polyethylenglykols an die anorganischen Träger angebunden werden.

Das Verfahren zur Herstellung von erfindungsgemäßem bioaktiven Hydrogel ist dadurch charakterisiert, dass
a) die Komponenten Heparin,
   1-Ethyl-3-(3-dimethylaminopropyl) carbodiimid EDC, N-Hydroxysulfosuccinimid s-NHS und
   sternförmiges Polyethylenglykol star-PEG separat gelöst werden, wonach
b) EDC und s-NHS als Aktivierungsreagenzien für die Carboxylgruppen des Heparins gemischt werden, wobei
c) das Heparin durch Zugabe von EDC und s-NHS aktiviert wird und dass nachfolgend
d) star-PEG zugegeben und die Mischung homogenisiert wird und
e) die Gelbildung anschließend erfolgt, wobei
f) im Anschluss an die Gelbildung das fertig gebildete Gel gewaschen wird.

Eine vorteilhafte Ausgestaltung des Verfahrens besteht darin, dass
a) die Komponenten Heparin, 1-Ethyl-3-(3-dimethylaminopropyl) carbodiimid EDC, N-Hydroxysulfosuccinimid s-NHS und sternförmiges Polyethylenglykol star-PEG separat in deionisiertem Wasser bei 4 °C gelöst werden, wonach
b) EDC und s-NHS als Aktivierungsreagenzien für die Carboxylgruppen des Heparins im Verhältnis 2 zu 1 gemischt werden, wobei
c) die Aktivierung des Heparins nach Zugabe von EDC und s-NHS über 15 min bei 4 °C erfolgt und dass anschließend
d) star-PEG zugegeben und die Mischung bei 8 °C über 15 min homogenisiert wird und
e) die Gelbildung über einen Zeitraum von 1 bis 14 h bei Raumtemperatur erfolgt, wobei
f) im Anschluss das fertig gebildete Gel mit phosphatgepufferter Kochsalzlösung oder abwechselnd in sauren oder basischen Salzlösungen und in phosphatgepufferter Kochsalzlösung gewaschen werden.

Bevorzugt beträgt das Verhältnis von EDC und s-NHS bezogen auf die Aminogruppen des star-PEGs 1,75 zu 1, wobei das Verhältnis von sternförmigen Polyethylenglykol zu Heparin 1 zu 1 bis 6 zu 1 beträgt.

Das Verfahren zur Herstellung der Hydrogele wird dadurch vorteilhaft weitergebildet, dass das Hydrogel nach Verfahrensschritt f) mit einem Adhäsionsprotein modifiziert wird, wobei das gewaschene Hydrogel mit einer Lösung aus EDC/s-NHS in 1/15 M Phosphatpuffer mit pH = 5 über 30 min bei 4 °C aktiviert wird, wonach die Lösung gegen eine 0,2 mg/ml RGD-Peptid als Adhäsionsprotein enthaltende Lösung aus 100 mM Boratpuffer mit pH = 8 ausgetauscht und für 2 h bei Raumtemperatur immobilisiert wird, wonach das modifizierte Hydrogel nochmals mit PBS gespült wird.

Als RGD-Peptid wird vorteilhaft cycloRGDyK als Adhäsionsprotein eingesetzt.

Eine vorteilhafte Weiterentwicklung des Verfahrens besteht weiterhin darin, dass das Hydrogel mit den Wachstumsfaktoren basic Fibroblast Growth Factor b-FGF und Vascular Endothelial Growth Factor VEGF beladen wird, wobei die Hydrogele mit einer Lösung von b-FGF oder VEGF mit einer Konzentration von 1-5µg/ml in PBS über 4 bis 24 h bei Raumtemperatur inkubiert und anschließend in PBS gewaschen werden.

Die Konzeption der Erfindung besteht darin, dass die gewünschten Eigenschaften der Hydrogele durch die Synthese eines Hybridmaterials aus einer biologisch aktiven Komponente - der natürlichen EZM-Komponente Heparin - und eines synthetischen, sternförmig verzweigten endgruppenfunktionalisierten Polyethylenglykol (star-PEG) realisiert werden. Diese beiden Bestandteile bilden die Gerüststruktur, das eigentliche Scaffold.

Je nach Anwendung werden diese beiden Hauptkomponenten direkt durch 1-Ethyl-3-(3-dimethylaminopropyl) carbodiimid/N-Hydroxysullfosuccinimid (EDC/s-NHS) aktivierte Carboxylgruppen des Heparins und den endständigen Aminogruppen des star-PEGs kovalent durch Amidbindungen verbunden.

Alternativ wird die analoge Kopplungschemie angewandt, um kurze enzymspaltbare Peptidsequenzen als Vernetzungsmolekül zwischen star-PEG und Heparin zu binden. Der Vorteil liegt hierbei in der Synthese eines kovalent miteinander verbundenen Netzwerkes aus star-PEG und Heparin, was lokal durch die Enzymaktivität einzelner Zellen durch Spaltung der Peptidbrücken abgebaut werden kann. Dadurch wird ein partieller Umbau und Ersatz der synthetischen Matrix durch von den Zellen sezerniertes Material möglich, außerdem besteht die Möglichkeit für die Zellen, in das Material einzuwandern und sich zu vereinigen, um therapeutisch wichtige Strukturen, beispielsweise kapillarförmige Blutgefäße, zu bilden.

Unabhängig von der Art der Vernetzung - entweder direkt zwischen Heparin und star-PEG oder über die spaltbaren Peptidsequenzen - lassen sich die physikalischen Eigenschaften des Scaffolds über einen weiten Bereich variieren.

Die Zelladhäsion der Materialien wird durch die gezielte Modifizierung des Heparins mit kurzen Peptidsequenzen, zum Beispiel durch integrinbindende Arginin-Glycin-Asparaginsäure-(RGD)-Sequenzen, gesteuert.

Die biofunktionelle Komponente, das Heparin, wird neben der Kontrolle der Zelladhäsion auch zur kontrollierten Bindung und Freisetzung von therapeutisch relevanten Signalmolekülen, beispielsweise Wachstumsfaktoren, eingesetzt. Die allgemein bekannte hohe Affinität des stark negativ geladenen Heparins zu einer Vielzahl von Wachstumsfaktoren, wie zum Beispiel bFGF und VEGF, ermöglicht die reversible und naturidentische elektrostatische Bindung und Freisetzung dieser für eine Vielzahl von regenerativen Prozessen wichtigen Signalmoleküle. Die Beladung und Freisetzung kann dabei in gewissem Maße über die Netzwerkstruktur, charakterisiert durch Maschenweite und Vernetzungsgrad der Hydrogelmaterialien, beeinflusst werden.

Besonders vorteilhaft ist, dass alle Komponenten außerdem vollständig biologisch abbaubar sind und dass PEG und Heparin für den therapeutischen Einsatz im Menschen beispielsweise von der amerikanischen Arzneimittelzulassungsbehörde "Food and Drug Administration" (FDA) zugelassen sind.

Die Vorteile der Erfindung lassen sich wie folgt zusammengefasst darstellen:

Als Kerneigenschaft und somit entscheidenden Vorteil gegenüber den derzeitig verfügbaren Materialien können die erfindungsgemäßen Hydrogele die als Nachteile des Standes der Technik benannten fehlenden, wichtigen Funktionen der EZM abbilden.

Das in seinen physikalischen Eigenschaften, wie beispielsweise Steifigkeit und Hydratisierung, über einen weiten Bereich variierbare Scaffold aus den erfindungsgemäßen Hydrogelen bildet die Gerüst-, Stütz- und Schutzfunktion für einwachsende Zellen. Die Materialien sind aufgrund des weiten Bereichs der Steifigkeit entweder minimalinvasiv durch Injektion oder als vorgefertigte Form- und Funktionskörper transplantierbar.

Die Variation der physikalischen Eigenschaften erfolgt gezielt über die Qualität und Quantität der Kopplungspunkte innerhalb des Netzwerkes der Hydrogele. Die Qualität der Kopplung wird dabei konzeptionsgemäß über die verwendeten Kopplungsmechanismen beziehungsweise über die modifizierten Komponenten determiniert, wohingegen die Quantität der Kopplung über die Verhältnisse der Komponenten zueinander und die Parameter der Aktivierung bestimmt wird.

Die allgemein bekannte Resistenz von PEG-Materialien gegenüber unspezifischer Proteinadsorption vermindert nicht gewünschte, unkontrollierte Wechselwirkungen mit Biomolekülen, währenddessen die Wechselwirkung mit den therapeutisch aktiven Zellen und Mikroorganismen durch die gezielte Modifizierung mit Zelladhäsionsproteinen (RGD-Peptiden) kontrolliert und geregelt werden kann. Die adäquate Präsentation des Heparins im Netzwerk ermöglicht die reversible Bindung und Freisetzung therapeutisch relevanter Signalmoleküle und die Möglichkeit des bedarfsgerechten Umbaus durch einwachsende Zellen wird über die Vernetzung durch enzymatisch spaltbare Peptidbrücken realisiert.

Ein wesentlicher Vorteil besteht zudem im modularen Charakter der Hydrogele. Neben der breiten Variation der physikalischen Eigenschaften über den Vernetzungsgrad ist auch die Biofunktionalisierung modular möglich, so sind zum Beispiel die Kontrolle der Zelladhäsion über RGD-Peptide, die direkte Vernetzung oder die Vernetzung mit enzymspaltbaren Peptiden und die Beladung mit Wachstumsfaktoren unabhängig voneinander und in weiten Bereichen variierbar.

Aus den Eigenschaften ergibt sich, dass die bioaktiven Hydrogele langfristig in Zeiträumen von mehreren Wochen bis Monaten einsetzbar sind.

Je nach Einsatzgebiet können über die Variation der Verhältnisse der Komponenten zueinander und die Variation der Verfahrensparameter für unterschiedliche Gewebsarten unterschiedliche physikalische Eigenschaften zielgerichtet erzeugt werden, um die körpereigenen anzulagernden oder einzuwandernden Zellen adäquat zu kultivieren. Dies wird besonders deutlich, wenn beispielsweise die Anforderungen an Nerven- oder Muskelzellen betrachtet werden. Aus diesem Grund ist es vorteilhaft, wenn die Implantatmaterialien über einen großen Bereich abstufbare Eigenschaften aufweisen.

Im Unterschied zum Stand der Technik ist die Kombination der Möglichkeit des Umbauens der molekularen Strukturen durch einwachsende Zellen und die natürliche Bindung der Signalmoleküle, insbesondere der Wachstumsfaktoren, an die bioaktive Komponente Heparin ein entscheidender Entwicklungsschritt. Der Vorteil wird bei den erfindungsgemäßen Hydrogelen dadurch erreicht, dass die Möglichkeit zum bedarfsgerechten Umbau durch einwandernde Zellen analog zu den natürlichen Mechanismen in der EZM gegeben ist, da die Signalmoleküle reversibel gebunden sind.

Besonders bevorzugte Anwendungsgebiete der erfindungsgemäßen Hydrogele sind der Einsatz als Implantatmaterialien in regenerativen Therapien, beispielsweise zur Unterstützung der Regeneration von Blutgefäßen mittels injizierbarer Gele, für Nervenbahnen sowohl des zentralen als auch peripheren Nervensystems als tubuläre Strukturen und als temporäres Hornhautersatzmaterial beim Einsatz von Folien.

Weitere Einzelheiten, Merkmale und Vorteile der Erfindung ergeben sich aus der nachfolgenden Beschreibung von Ausführungsbeispielen mit Bezugnahme auf die zugehörige Zeichnung.

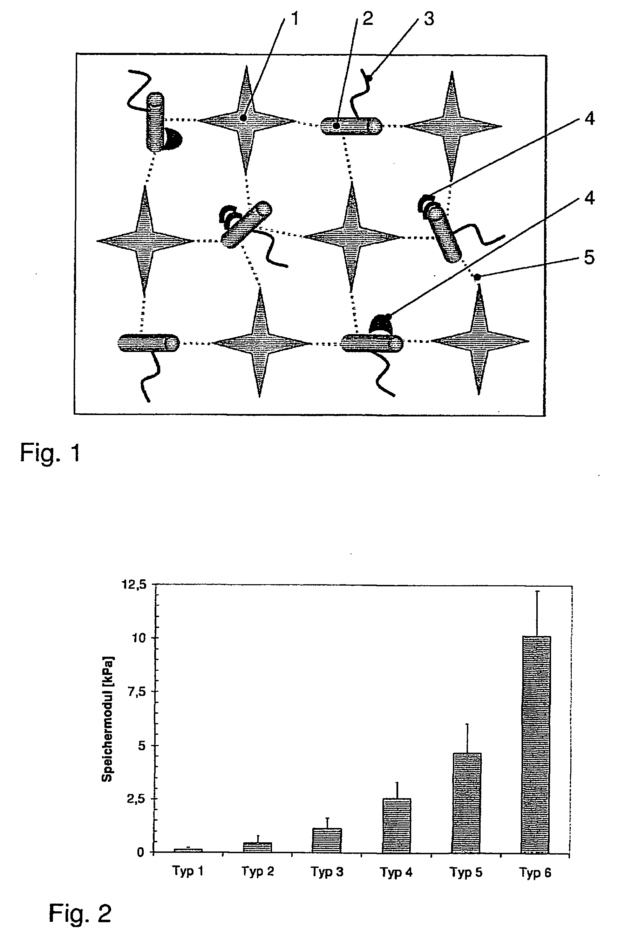

In Fig. 1 ist eine schematische Darstellung des synthetischen Hydrogels aus Heparin und amino-endfunktionalisiertem, sternförmig verzweigtem Polyethylenglykol abgebildet.

Das sternförmig verzweigte, amino-endfunktionalisierte Polyethylenglykol 1 ist über enzymatisch spaltbare Peptidsequenzen 5 jeweils mit Heparinmolekülen 2 netzwerkartig verbunden. Die Heparinmoleküle 2 weisen weiterhin RGD-Peptide 3, also intigrinbindende Arginin-Glycin-Asparginsäure-(RGD)-Sequenzen auf. Schematisch sind weiterhin verschiedene am Heparin 2 angedockte Signalmoleküle 4 dargestellt.

Das Basisverfahren zur Herstellung von bioaktivem Hydrogel lässt sich wie folgt beschreiben:

Die Komponenten Heparin, EDC, s-NHS und star-PEG werden separat in deionisiertem Wasser auf Eis bei 4 °C gelöst. Das Verhältnis der Aktivierungsreagenzien für die Carboxylgruppen des Heparins beträgt EDC zu s-NHS gleich 2 zu 1. Nach der Auflösung wird das EDC/s-NHS zum Heparin gegeben und die Carboxylgruppen des Heparins 15 min bei 4 °C aktiviert. Im Anschluss wird das star-PEG zugegeben und die Mischung bei 8 °C 15 min homogenisiert (bei 900 u/min, Thermomixer Comfort, Eppendorf, Hamburg, Germany). Im Anschluss erfolgt die weitere Gelbildung über einen Zeitraum von 1 bis 14 h bei Raumtemperatur, gefolgt von mehrmaligen (mindestens 5-mal, für einen Zeitraum von 1 h) Waschschritten in PBS - alternativ werden die fertigen Gele abwechselnd in sauren oder basischen Salzlösungen und in PBS gespült.

Alternativ wird mit enzymspaltbaren Peptiden funktionalisiertes star-PEG oder Heparin zum Vernetzen angewendet; die technische Prozedur ist analog zum zuvor beschriebenen Verfahren.

Der vorangehend beschriebene Basisverfahrensablauf ist nun jeweils unter spezifischen Rahmenbedingungen modifizierbar, um gezielt bestimmte Eigenschaften der Hydrogele zu erzeugen.

(A) Es werden Hydrogele nach dem Basisverfahren durch direkte Umsetzung von EDC (Sigma-Aldrich, München, Germany) /s-NHS (Sigma-Aldrich, München, Germany) aktivierten Carboxylgruppen des Heparins (MW 14.000, Calbiochem (Merck), Darmstadt, Germany) mit den Aminogruppen des star-PEGs (MW 10.000, Polymer Source, Inc., Dorval, Canada) synthetisiert. EDC/s-NHS wird bezogen auf die Aminogruppen des star-PEGs im Verhältnis EDC 1,75 zu 1 Aminogruppen eingesetzt.

Das Verhältnis zwischen Heparin und star-PEG entscheidet dabei über den Vernetzungsgrad und somit über die physikalischen Eigenschaften der resultierenden Gelmaterialien. Hierfür werden molare Verhältnisse von star-PEG zu Heparin von 1 zu 1 bis 6 zu 1 eingesetzt. Von diesen molaren Verhältnissen abgeleitet ist die Bezeichnung der Gele in Fig. 2 mit Typ 1 bis Typ 6.

Die aus der Variation der Verhältnisse resultierenden physikalischen Eigenschaften sind anhand der Darstellung in Fig. 2 signifikant ableitbar.

(B) Nach einer weiteren Ausgestaltung der Erfindung werden Hydrogele nach dem Basisverfahren hergestellt, die sich von der Ausgestaltung unter (A) durch das eingesetzte Heparin unterscheiden. An Stelle von Heparin 14.000 MW wird ein Heparin mit kürzerer Kettenlänge von 4.000 MW (Sigma-Aldrich, München, Germany) eingesetzt.

(C) Bei dieser Ausgestaltung der Erfindung werden Hydrogele nach dem Basisverfahren hergestellt, die sich von (A) wiederum durch das eingesetzte Heparin unterscheiden. An Stelle von unmodifiziertem Heparin 14.000 MW wird ein mit enzymatisch spaltbaren Peptidsequenzen modifiziertes Heparin eingesetzt. Die enzymatisch spaltbaren Peptidsequenzen sind nach dem Ein-Buchstaben-Code: GPQG↓IAGQ oder GPQG↓IWGQ charakterisiert und werden durch Festphasenpeptidsynthese hergestellt.

(D) In dieser Ausführungsform wird das nach dem Basisverfahren hergestellte Hydrogel, das sich von der Ausgestaltung unter (A) gleichfalls durch das eingesetzte Heparin unterscheidet. An Stelle von unmodifiziertem Heparin 14.000 MW wird ein mit Adhäsionsprotein (Sequenz: cycloRGDyK, Peptides International, Louisville, KY, USA) modifiziertes Heparin eingesetzt.

(E) Das Hydrogel wird wiederum nach dem Basisverfahren hergestellt, wobei sich die Ausgestaltungen (A bis D) durch das eingesetzte star-PEG unterscheiden. Hierbei wird an Stelle von star-PEG 10.000 MW ein star-PEG der Molmasse 19.000 MW (Polymer Source, Inc., Dorval, Canada) eingesetzt.

Eine Variante dieser Ausgestaltung besteht darin, dass an Stelle von unmodifiziertem PEG 10.000 MW/19.000 MW ein - mit enzymatisch spaltbaren Peptidsequenzen - modifiziertes star-PEG eingesetzt wird. Die enzymatisch spaltbaren Peptidsequenzen sind nach dem Ein-Buchstaben-Code: GPQG↓IAGQ oder GPQG↓IWGQ charakterisiert und werden durch Festphasenpeptidsynthese hergestellt.

(F) Nach einer weiteren Ausgestaltung werden die Hydrogele nach den Ausführungen (A, B, C und E) hergestellt, die nach dem Waschschritt nachträglich mit Adhäsionsproteinen (allgemein RGD-Peptid, Beispiel cycloRGDyK) modifiziert werden. Hierzu werden die gewaschenen Gelmaterialien mit einer Lösung aus EDC/s-NHS in 1/15 M Phosphatpuffer (pH = 5) 30 min bei 4 °C aktiviert. Im Anschluss wird die Lösung gegen eine 0,2 mg/ml (cycloRGDyK) enthaltende Lösung aus 100 mM Boratpuffer (pH = 8) ausgetauscht und für 2 h bei Raumtemperatur immobilisiert. Als finalen Schritt werden die Materialien wieder mehrfach mit PBS gespült.

(G) Sämtliche Hydrogele der Ausgestaltungen (A bis F) werden nach einer weiteren vorteilhaften Ausgestaltung anschließend mit Wachstumsfaktoren (b-FGF oder VEGF) beladen. Hierzu wurden die Gelmaterialien mit b-FGF oder VEGF (1-5µg/ml) gelöst in PBS 4 bis 24 h bei Raumtemperatur inkubiert. Anschließend wurden die Materialien in PBS gewaschen.

(H) Die Hydrogele der Typen 1, 2, 3 und 4 sind aufgrund des geringeren Vernetzungsgrades durch eine handelsübliche Spritze der Gauge-Größe 27 injizierbar.

(I) Die Hydrogele der Typen 5 und 6 nach den Ausführungsbeispielen (A bis G) sind aufgrund Ihrer Steifigkeit auch zur Herstellung von Formkörpern geeignet. Es sind tubuläre Strukturen von bis zu 7 cm Länge und einem Innendurchmesser von 0,3 bis 0,8 mm erzeugbar.

(J) Eine weitere Ausgestaltung zur Erzeugung tubulärer Strukturen werden in Form eines Hybridsystemes aus einem Schlauch und Gelmaterialien der Typen 1 bis 6 gemäß der Ausführungsbeispiele (A bis G) erzeugt. Hierfür werden in einen kapillarförmigen Schlauch (zum Beispiel aus rekonstituierter Zellulose) mit Innendurchmessern im Bereich von 0,3 bis 0,8 mm durch Injektion mit den fertig gemischten, noch flüssigen Gelmaterialien gefüllt und anschließend ein Platzhalter, zum Beispiel ein Glasstab mit einem Außendurchmesser im Bereich von 0,2 bis 0,5 mm in das Innere eingeführt. Nach der Gelbildung werden die Hybridsysteme gequollen und der Platzhalter im Inneren wird entfernt. Es entstehen Hybridtubes aus einem Schlauchträgermaterial und dem Hydrogel im Inneren. Der Vorteil dieses Hybridsystems liegt in der Möglichkeit, alle Geltypen im Inneren zu verwenden, auch Geltypen mit geringem Speichermodul beziehungsweise geringer Steifigkeit.

(K) Eine weitere alternative Anwendung der Hydrogele liegt bei Folien der Stärke von etwa 80 bis 2000 µm. Durchmesser von 15 bis 25 mm werden aus den Gelen der Typen 5 oder 6 der Ausführungsbeispiele (A bis G) durch Auftropfen einer definierten Menge flüssiger Gelmaterialen auf hydrophobierte Deckgläser oder Siliciumwafer hergestellt, die im Anschluss wieder durch hydrophobierte Glasträger/Siliciumwafer abgedeckt werden. Nach der Gelbildung werden die Glasträger in PBS überführt und können so leicht durch Quellen entfernt werden.

(L) Die Gelmaterialien der Typen 1 bis 6 der Ausführungsbeispiele (A bis G) wurden für 2D-Zellkulturträger kovalent über reaktive Polymerdünnschichten (alternierende MSA-Copolymere) angekoppelt. Hierzu wird die Gelbildung in Gegenwart der Anhydridgruppen enthaltenden polymerbeschichteten anorganischen Träger durchgeführt und somit die Gelmaterialien über die Aminogruppen des star-PEGs an die anorganischen Träger angebunden.

Die Vorteile und die Wirksamkeit der offenbarten Ausgestaltungen wurden experimentell verifiziert.

Die nach den verschiedenen Ausgestaltungen der Erfindung erhaltenen Materialien erlauben aufgrund der Art der chemischen Bindung die Abstufung der physikalischen Eigenschaften über einen weiten Bereich. Das Speichermodul als ein Kernparameter zur Beschreibung der Steifigkeit der Materialien und der Quellungsgrad der Materialien ist je nach Vernetzungsgrad über einen weiten Bereich durch Variation einzelner Parameter abstufbar. In Figur 2 ist das Speichermodul in Abhängigkeit des Typs der einzelnen Ausgestaltungen von Hydrogelen dargestellt.

Das Speichermodul wurde mit oszillierenden Messungen an gequollenen Gelen in PBS an einem rotierenden Rheometer der Firma ARES (ARES LN2, TA Instruments, Eschbom, Germany) bestimmt. Als Geometrie kam eine Platte/Platte-Anordnung zum Einsatz (Plattendurchmesser 25 mm, der Spalt dazwischen lag in einem Bereich von 1,2 bis 1,5 mm). Die Messungen wurden bei 25 °C in einem Frequenzbereich von 10+2 - 10-1 rad×s-1 ausgeführt. Die Amplitude der Deformation wurde auf 3 % gesetzt. Das Speichermodul wurde als eine Funktion der Scherfrequenz gemessen. Die Mittelwerte des Speichermoduls im Frequenzbereich zwischen 100 - 101 rad×s-1 wurden aus mindestens 3 unabhängigen Messungen ermittelt.

### LISTE DER BEZUGSZEICHEN

- 1: sternförmiges Polyethylenglykol, (PEG), star-PEG
- 2: Heparin
- 3: integrinbindende Arginin-Glycin-Asparaginsäure-(RGD)-Sequenzen
- 4: Signalmoleküle
- 5: enzymatisch spaltbare Peptidsequenzen

### Abkürzungen in der Beschreibung

- EZM: Extrazelluläre Matrix
- EDC: 1-Ethyl-3-(3-dimethylaminopropyl) carbodiimid
- s-NHS: N-Hydroxysulfosuccinimid
- bFGF: basic Fibroblast Growth Factor
- VEGF: Vascular Endothelial Growth Factor
- PBS: phosphatgepufferte Kochsalzlösung
- PEG: Polyethylenglykol

## Patentansprüche

1. Bioaktives Hydrogel als Hybridmaterial aus Heparin und sternförmig verzweigtem endgruppenfunktionalisierten Polyethylenglykol, wobei das Heparin direkt durch Reaktion der mit 1-Ethyl-3-(3-dimethylaminopropyl) carbodiimid/N-Hydroxysulfosuccinimid (EDC/s-NHS) aktivierten Carboxylgruppen mit den endständigen Aminogruppen des Polyethylenglykols kovalent durch Amidbindungen verbunden ist.

2. Bioaktives Hydrogel als Hybridmaterial aus Heparin und sternförmig verzweigtem endgruppenfunktionalisierten Polyethylenglykol, wobei das Heparin über kurze enzymspaltbare Peptidsequenzen als Vernetzungsmolekül kovalent mit dem Polyethylenglykol verbunden ist.

3. Bioaktives Hydrogel nach Anspruch 2, **dadurch gekennzeichnet, dass** die Funktionalisierung des Polyethylenglykols mit enzymspaltbaren Peptidsequenzen durch Reaktion der mit 1-Ethyl-3-(3-dimethylaminopropyl) carbodiimid/N-Hydroxysulfosuccinimid (EDC/s-NHS) aktivierten Carboxylgruppe am C-Terminus des Peptides mit den Aminogruppen des Polyethylenglykols erfolgt und dass im Anschluss das Gel durch die Umsetzung der Aminogruppe am N-Terminus des mit dem PEG verbundenen Peptides mit den mit 1-Ethyl-3-(3-dimethylaminopropyl) carbodiimid/N-Hydroxysulfosuccinimid (EDC/s-NHS) aktivierten Carboxylgruppen des Heparins gebildet ist.

4. Bioaktives Hydrogel nach Anspruch 2, **dadurch gekennzeichnet, dass** die Funktionalisierung des Heparins mit enzymspaltbaren Peptidsequenzen durch Reaktion der mit 9-Ethyl-3-(3-dimethylaminopropyl) carbodiimid/N-Hydroxysulfosuccinimid (EDC/s-NHS) aktivierten Carboxylgruppen des Heparins mit der Aminogruppe am N-Terminus des Peptides erfolgt und dass im Anschluss das Gel durch Umsetzung des mit den Peptiden funktionalisierten Heparins gebildet wird, indem die durch 1-Ethyl-3-(3-dimethylaminopropyl) carbodiimid/N-Hydroxysulfosuccinimid (EDC/s-NHS) aktivierte Carboxylgruppe am C-Terminus des Peptides mit den Aminogruppen des PEGs reagiert.

5. Bioaktives Hydrogel nach einem der Ansprüche 2 bis 4, **dadurch gekennzeichnet, dass** als kurze Peptidsequenz integrinbindende Arginin-Glycin-Asparaginsäure eingesetzt ist.

6. Bioaktives Hydrogel nach einem der Ansprüche 1 bis 5, **dadurch gekennzeichnet, dass** Signalmoleküle an das Heparin reversibel elektrostatisch angekoppelt sind.

7. Bioaktives Hydrogel nach Anspruch 6, **dadurch gekennzeichnet, dass** als Signalmoleküle Wachstumsfaktoren an das Heparin reversibel elektrostatisch angekoppelt sind.

8. Bioaktives Hydrogel nach Anspruch 7, **dadurch gekennzeichnet, dass** als Wachstumsfaktoren bFGF oder VEGF an das Heparin reversibel elektrostatisch angekoppelt sind.

9. Bioaktives Hydrogel nach einem der Ansprüche 1 bis 8, **dadurch gekennzeichnet, dass** Heparin mit einer Kettenlänge von 4.000 bis 14.000 MW eingesetzt ist.

10. Bioaktives Hydrogel nach einem der Ansprüche 1 bis 9, **dadurch gekennzeichnet, dass** star-PEG mit einer Molmasse von 10.000 bis 19.000 MW eingesetzt ist.

11. Bioaktives Hydrogel nach einem der Ansprüche 1 bis 10, **dadurch gekennzeichnet, dass** als Heparin ein mit enzymatisch spaltbaren Peptidsequenzen der Form GPQG↓IAGQ oder GPQG↓IWGQ modifiziertes Heparin eingesetzt ist.

12. Bioaktives Hydrogel nach einem der Ansprüche 1 bis 11, **dadurch gekennzeichnet, dass** als Heparin ein mit dem Adhäsionsprotein der Sequenz cycloRGDyK modifiziertes Heparin eingesetzt ist.

13. Folien aus Hydrogelen nach einem der Ansprüche 1 bis 12, erhältlich durch
- Auftropfen einer definierten Menge flüssiger Gelmaterialien auf hydrophobierte Trägerfläche,
- Gelbildung nach dem Abdecken der Trägerflächen mit hydrophobierten Abdeckfläche
- Überführen der Trägerflächen in eine Waschlösung und
- Entfernung der Folien nach dem Quellen der Hydrogele von den Trägerflächen.

14. Zellkulturträger mit einem Hydrogel nach einem der Ansprüche 1 bis 12,
**dadurch gekennzeichnet, dass**
das Hydrogel kovalent über reaktive Polymerdünnschichten aus alternierenden MSA-Copolymeren angekoppelt ist, wobei die Gelbildung in Gegenwart der Anhydridgruppen enthaltenden polymerbeschichteten anorganischen Träger durchgeführt wird und die Hydrogele über die Aminogruppen des star-PEGs an die anorganischen Träger gebunden sind.

15. Verfahren zur Herstellung von bioaktivem Hydrogel nach einem der Ansprüche 1 bis 12, bei dem
a) die Komponenten Heparin,
1-Ethyl-3-(3-dimethylaminopropyl) carbodiimid EDC, N-Hydroxysulfosuccinimid s-NHS und
sternförmiges Polyethylenglykol star-PEG separat gelöst werden, wonach
b) EDC und s-NHS als Aktivierungsreagenzien für die Carboxylgruppen des Heparins gemischt werden, wobei
c) das Heparin durch Zugabe von EDC und s-NHS aktiviert wird und dass nachfolgend
d) star-PEG zugegeben und die Mischung homogenisiert wird und
e) die Gelbildung anschließend erfolgt, wobei
f) im Anschluss das fertig gebildete Gel gewaschen wird.

16. Verfahren nach Anspruch 15, **dadurch gekennzeichnet, dass**
a) Heparin,
1-Ethyl-3-(3-dimethylaminopropyl) carbodiimid EDC, N-Hydroxysulfosuccinimid s-NHS und
sternförmiges Polyethylenglykol star-PEG separat in deionisiertem Wasser bei 4 °C gelöst werden, wonach
b) EDC und s-NHS als Aktivierungsreagenzien für die Carboxylgruppen des Heparins im Verhältnis von zwei zu eins gemischt werden, wobei
c) die Aktivierung des Heparins nach Zugabe von EDC und s-NHS über 15 min bei 4 °C erfolgt und dass anschließend
d) star-PEG zugegeben und die Mischung bei 8 °C über 15 min homogenisiert wird und
e) die Gelbildung über einen Zeitraum von 1 bis 14 h bei Raumtemperatur erfolgt, wobei
f) im Anschluss das fertig gebildete Gel mit phosphatgepufferter Kochsalzlösung oder abwechselnd in sauren oder basischen Salzlösungen und in phosphatgepufferter Kochsalzlösung gewaschen werden.

17. Verfahren nach Anspruch 15 oder 16, **dadurch gekennzeichnet, dass** das Verhältnis von EDC und s-NHS bezogen auf die Aminogruppen des star-PEGs 1,75 zu 1 beträgt, wobei das Verhältnis von star-PEG zu Heparin 1 zu 1 bis 6 zu 1 beträgt.

18. Verfahren nach einem der Ansprüche 15 bis 17, **dadurch gekennzeichnet, dass** das Hydrogel nach Verfahrensschritt f) mit dem Adhäsionsproteinen cycloRGDyK modifiziert werden, wobei die gewaschenen Hydrogele mit einer Lösung aus EDC/s-NHS in 1/15 M Phosphatpuffer mit pH = 5 über 30 min bei 4 °C aktiviert werden, wonach die Lösung gegen eine 0,2 mg/ml cycloRGDyK enthaltende Lösung aus 100 mM Boratpuffer mit pH = 8 ausgetauscht und für 2 h bei Raumtemperatur immobilisiert wird, wonach das modifizierte Hydrogel nochmals mit PBS gespült wird.

19. Verfahren nach einem der Ansprüche 15 bis 18, **dadurch gekennzeichnet, dass** das Hydrogel mit Wachstumsfaktoren b-FGF oder VEGF beladen wird, wobei die Hydrogele mit einer Lösung von b-FGF oder VEGF mit einer Konzentration von 1-5µg/ml in PBS über 4 bis 24 h bei Raumtemperatur inkubiert und anschließend in PBS gewaschen werden.

## Claims

1. A bioactive hydrogel acting as a hybrid material, formed from heparin and starbranched polyethylene glycol having functionalized end groups, wherein the heparin is directly covalently bonded via amide bonds formed by reacting the carboxyl groups activated with 1-ethyl-3-(3-dimethylaminopropyl) carbodiimide/N-hydroxysulphosuccinimide (EDC/s-NHS) with the terminal amino groups of the polyethylene glycol.

2. A bioactive hydrogel acting as a hybrid material, formed from heparin and starbranched polyethylene glycol having functionalized end groups, wherein the heparin is covalently bonded with the polyethylene glycol via short enzyme-cleavable peptide sequences acting as cross-linking molecules.

3. The bioactive hydrogel as claimed in claim 2, **characterized in that** functionalization of the polyethylene glycol with enzyme-cleavable peptide sequences is carried out by reacting the carboxyl group at the C-terminus of the peptide, activated with 1-ethyl-3-(3-dimethylaminopropyl) carbodiimide/N-hydroxysulphosuccinimide (EDC/s-NHS), with the amino groups of the polyethylene glycol, and **in that** the gel is then formed by reacting the amino group at the N-terminus of the peptide bonded to the PEG with the carboxyl groups of the heparin activated with 1-ethyl-3-(3-dimethylaminopropyl) carbodiimide/N-hydroxysulphosuccinimide (EDC/s-NHS).

4. The bioactive hydrogel as claimed in claim 2, **characterized in that** functionalization of the heparin with enzyme-cleavable peptide sequences is carried out by reacting the carboxyl groups of the heparin activated with 1-ethyl-3-(3-dimethylaminopropyl) carbodiimide/N-hydroxysulphosuccinimide (EDC/s-NHS) with the amino group at the N-terminus of the peptide and **in that** the gel is then formed by reacting the peptide-functionalized heparin wherein the carboxyl group at the C-terminus of the peptide activated with 1-ethyl-3-(3-dimethylaminopropyl) carbodiimide/N-hydroxysulphosuccinimide (EDC/s-NHS) is reacted with the amino groups of the PEG.

5. The bioactive hydrogel as claimed in one of claims 2 to 4, **characterized in that** integrin-binding arginine-glycine-aspartic acid is used as the short peptide sequence.

6. The bioactive hydrogel as claimed in one of claims 1 to 5, **characterized in that** signal molecules are reversibly and electrostatically coupled to the heparin.

7. The bioactive hydrogel as claimed in claim 6, **characterized in that** growth factors acting as signal molecules are reversibly and electrostatically coupled to the heparin.

8. The bioactive hydrogel as claimed in claim 7, **characterized in that** bFGF or VEGF acting as growth factors are reversibly and electrostatically coupled to the heparin.

9. The bioactive hydrogel as claimed in one of claims 1 to 8, **characterized in that** heparin with a chain length of 4 000 to 14 000 MW is used.

10. The bioactive hydrogel as claimed in one of claims 1 to 9, **characterized in that** star-PEG with a molecular weight of 10 000 to 19 000 MW is used.

11. The bioactive hydrogel as claimed in one of claims 1 to 10, **characterized in that** the heparin employed is a heparin modified with an enzymatically cleavable peptide sequence with the form GPQG↓IAGQ or GPQG↓IWGQ.

12. The bioactive hydrogel as claimed in one of claims 1 to 11, **characterized in that** the heparin employed is a heparin modified with the adhesion protein with the sequence cycloRGDyK.

13. Films formed from hydrogels as claimed in one of claims 1 to 12, obtainable as follows:
• applying a predetermined amount of liquid gel materials dropwise onto support surfaces that have been rendered hydrophobic;
• forming a gel after covering the support surfaces with cover surfaces that have been rendered hydrophobic;
• transferring the support surfaces to a washing solution; and
• removing the films from the support surfaces after swelling the hydrogels.

14. A cell culture support having a hydrogel as claimed in one of claims 1 to 12, **characterized in that** the hydrogel is covalently coupled via thin layers of reactive polymers formed from alternating MSA copolymers, wherein formation of the gel is carried out in the presence of polymer-coated inorganic carriers containing anhydride groups and the hydrogels are bonded to the inorganic support via the amino groups of the star-PEG.

15. A method for producing bioactive hydrogel as claimed in one of claims 1 to 12,
wherein
a) the components heparin, 1-ethyl-3-(3-dimethylaminopropyl) carbodiimide, EDC, N-hydroxysulphosuccinimide, s-NHS, and star-shaped polyethylene glycol, star-PEG, are dissolved separately, after which
b) EDC and s-NHS, acting as activation reagents for the carboxyl groups of heparin, are mixed together; wherein
c) the heparin is activated by adding EDC and s-NHS; and then
d) star-PEG is added and the mixture is homogenized; and
e) gel formation is then carried out; wherein
f) next, the finished formed gel is washed.

16. The method as claimed in claim 15, **characterized in that**
a) heparin, 1-ethyl-3-(3-dimethylaminopropyl) carbodiimide, EDC, N-hydroxysulphosuccinimide, s-NHS, and star-shaped polyethylene glycol, star-PEG, are dissolved separately in deionized water at 4°C; after which
b) EDC and s-NHS are mixed together as activation reagents for the carboxyl groups of the heparin in a ratio of two to one; wherein
c) after adding EDC and s-NHS, the heparin is activated for 15 min at 4°C; and then
d) star-PEG is added and the mixture is homogenized at 8°C for 15 min; and
e) gel formation is carried out for a period of 1 to 14 h at room temperature; whereupon
f) the finished formed gel is then washed with phosphate-buffered sodium chloride solution or is washed alternately in acid or basic salt solutions and in phosphate-buffered sodium chloride solution.

17. The method as claimed in claim 15 or claim 16, **characterized in that** the ratio of the EDC to the s-NHS with respect to the amino groups of the star-PEG is 1.75 to 1, whereas the ratio of the star-PEG to the heparin is from 1 to 1 to 6 to 1.

18. The method as claimed in one of claims 15 to 17, **characterized in that** after step f) of the method, the hydrogel is modified with the adhesion protein cycloRGDyK, wherein the washed hydrogels are activated with a EDC/s-NHS solution in 1/15 M phosphate buffer at a pH of 5 for 30 min at 4°C, after which the solution is replaced by a solution of 100 mM borate buffer at a pH of 8 containing 0.2 mg/mL cycloRGDyK and is immobilized over 2 h at room temperature, after which the modified hydrogel is rinsed again with PBS.

19. The method as claimed in one of claims 15 to 18, **characterized in that** the hydrogel is loaded with growth factors b-FGF or VEGF, wherein the hydrogels are incubated with a solution of b-FGF or VEGF in a concentration of 1-5 µg/mL in PBS for 4 to 24 h at room temperature and are then washed in PBS.

## Revendications

1. Hydrogel bioactif se présentant sous forme d'd'un matériau hybride composé d'héparine et de polyéthylène glycol fonctionnalisé par groupe cible et ramifié en étoile, l'héparine étant liée directement de manière covalente par réaction des groupes carboxyle activés par du 1-éthyle-3-(3-diméthylaminopropyl) carbodiimide-N-hydroxysulfo-succinimide (EDC/s-NHS) aux groupes aminés de fin de chaîne du polyéthylène glycol par des liaisons amide.

2. Hydrogel bioactif se présentant sous forme d'un matériau hybride composé d'héparine et de polyéthylène glycol fonctionnalisé par groupe cible et ramifié en étoile, l'héparine étant liée en tant que molécule de réticulation de manière covalente par des séquences peptide courtes scindables par enzyme au polyéthylène glycol.

3. Hydrogel bioactif selon la revendication 2, **caractérisé en ce que** la fonctionnalisation du polyéthylène glycol avec des séquences peptide scindables par enzyme se fait par réaction du groupe carboxyle activé par du 1-éthyle-3-(3-diméthylaminopropyl) carbodiimide-N-hydroxysulfo-succinimide (EDC/s-NHS) au terminus C du peptide avec les groupes aminés du polyéthylène glycol et que le gel est ensuite formé par la mutation du groupe aminé au terminus N du peptide relié au PEG avec les groupes carboxyle activés par du 1-éthyle-3-(3-diméthylaminopropyl)carbodiimide- N-hydroxysulfo-succinimide (EDC/s-NHS) de l'héparine.

4. Hydrogel bioactif selon la revendication 2, **caractérisé en ce que** la fonctionnalisation de l'héparine avec des séquences peptide scindables par enzyme se fait par réaction des groupes carboxyle activés par du 1-éthyle-3-(3-diméthylaminopropyl) carbodi-imide-N-hydroxysulfo-succinimide (EDC%s-NHS) de l'héparine avec le groupe aminé au terminus N du peptide et que, ensuite, le gel réagit par conversion de l'héparine fonctionnalisée avec les peptides du fait que le groupe carboxyle activé par du 1-éthyle-3-(3-diméthylaminopropyl) carbodiimide-N-hydroxysulfo-succinimide (EDC/s-NHS) réagit au terminus C du peptide avec les groupes aminés du PEG.

5. Hydrogel bioactif selon une des revendications 2 à 4, **caractérisé en ce qu'**on utilise comme séquence peptide courte de l'acide arginique-glycinique-asparaginique liant l'intégrine.

6. Hydrogel bioactif selon une des revendications 1 à 5, **caractérisé en ce que** des molécules de signalisation sont couplées électrostatiquement de manière réversible à l'héparine.

7. Hydrogel bioactif selon la revendication 6, **caractérisé en ce que** des facteurs de croissance faisant office de molécules de signalisation sont couplés électrostatiquement de manière réversible à l'héparine.

8. Hydrogel bioactif selon la revendication 7, **caractérisé en ce que** des bFGF ou VEGF sont couplés électrostatiquement de manière réversible à l'héparine.

9. Hydrogel bioactif selon une des revendications 1 à 8, **caractérisé en ce que** l'héparine est utilisée avec une longueur de chaîne de 4 000 à 14 000 MV.

10. Hydrogel bioactif selon une des revendications 1 à 9, **caractérisé en ce que** du PEG star ayant une masse molaire de 10 000 à 19 000 MV est utilisé.

11. Hydrogel bioactif selon une des revendications 1 à 10, **caractérisé en ce qu'**on utilise comme héparine une héparine modifiée avec des séquences peptide scindables par enzyme de forme GPQG↓IAGQ ou GPQG↓IWGQ.

12. Hydrogel bioactif selon une des revendications 1 à 11, **caractérisé en ce qu'**on utilise comme héparine une héparine modifiée avec la protéine d'adhérence de séquence cycloRGDyK.

13. Films à base d'hydrogel selon une des revendications 1 à 12, pouvant être obtenus en
- faisant goutter une quantité définie de matériaux gélifiés liquides sur des surfaces supports rendues hydrophobes,
- constituant du gel après le recouvrement des surfaces supports par des surfaces couvrantes rendues hydrophobes,
- mettant les surfaces supports dans une solution de lavage et
- enlevant les films des surfaces supports après gonflement des hydrogels.

14. Support de culture cellulaire contenant un hydrogel selon une des revendications 1 à 12, **caractérisé en ce que** l'hydrogel est couplé de manière covalente par des couches minces réactives de polymère à base de copolymères MSA alternants, la formation du gel étant réalisée en présence des supports organiques revêtus de polymère contenant des groupes anhydride, et que les hydrogels sont liés par les groupes aminés du PEG star aux supports anorganiques.

15. Procédé de fabrication d'hydrogel bioactif selon une des revendications 1 à 12, dans lequel
a) les composants héparine, 1-éthyle-3-(3-diméthylaminopropyl)carbodiimide EDC, N-hydroxysulfo-succinimide s-NHS) et le polyéthylène glycol PEG star sont dissous séparément, après quoi
b) l'EDC et le s-NHS sont mélangés en tant que réactifs d'activation pour les groupes carboxyle de l'héparine,
c) l'héparine étant activée par ajout d'EDC et de s-NHS et que, ensuite,
d) du PEG star est ajouté et le mélange est homogénéisé et
e) que la formation de gel a ensuite lieu,
f) le gel ayant fini de se former étant ensuite lavé.

16. Procédé de fabrication d'hydrogel bioactif selon la revendication 15, **caractérisé en ce que**
a) l'héparine, le 1-éthyle-3-(3-diméthylaminopropyl) carbodiimide EDC, le N-hydroxysulfo-succinimide s-NHS et le polyéthylène glycol PEG star en étoile sont dissous séparément dans de l'eau déionisée à 4° C, après quoi
b) l'EDC et le s-NHS sont mélangés en tant que réactifs d'activation pour les groupes carboxyle de l'héparine dans un rapport de deux à un,
c) l'activation de l'héparine se faisant par ajout d'EDC et de s-NHS pendant 15 min à 4° C et que, ensuite,
d) du PEG star est ajouté et le mélange est homogénéisé pendant 15 min à 8° C et que
e) la formation de gel a lieu sur une durée de 1 à 14 h à température ambiante,
f) le gel ayant fini de se former étant ensuite lavé avec une solution de sel de cuisine tamponnée au phosphate ou alternativement dans des solutions salines acides ou basiques et dans une solution de sel de cuisine tamponnée au phosphate.

17. Procédé selon la revendication 15 ou 16, **caractérisé en ce que** le ratio de l'EDC et du s-NHS par rapport aux groupes aminés du PEG star est de 1,75 à 1, la rapport du PEG star par rapport à l'héparine étant compris entre 1 à 1 et 6 à 1.

18. Procédé selon une des revendications 15 à 17, **caractérisé en ce que** l'hydrogel, après l'étape opératoire f), est modifié avec les protéines d'adhérence cycloRGDyK, les hydrogels lavés étant activés avec une solution d'EDC/s-NHS dans 1/15 M de tampon de phosphate à un pH = 5 pendant 30 min à 4° C, après quoi la solution est changée contre une solution contenant 0,2 mg/ml de cycloRGDyK et composée de 100 mM de tampon de borate à un pH = 8 et immobilisée pendant 2 h à température ambiante, après quoi le gel modifié est à nouveau lavé avec du PBS.

19. Procédé selon une des revendications 15 à 18, **caractérisé en ce que** l'hydrogel est chargé de facteurs de croissance b-FGF ou VEGF, les hydrogels étant incubés avec une solution de b-FGF ou VEGF à une concentration de 1 à 5 µg/ml dans du PBS pendant 4 à 24 h à température ambiante puis lavés dans du PBS.
